# EUROPEAN PATENT SPECIFICATION

(11) **EP 0 445 322 B1**
(45) Date of publication and mention of the grant of the patent: **31.01.1996**
(21) Application number: 90104278.8
(22) Date of filing: 06.03.1990
(51) Int. Cl.: A61B 17/225

(54) **Shock wave medical treatment apparatus with exchangeable imaging ultrasonic wave probe**
Medizinische Vorrichtung zur Stosswellenbehandlung mit auswechselbarer Ultraschallsonde
Dispositif de traitement médical par ondes de choc avec sonde ultrasonique interchangeable

(43) Date of publication of application: 11.09.1991
(73) Proprietor: KABUSHIKI KAISHA TOSHIBA, Kawasaki-shi, Kanagawa-ken 210, Tokyo (JP)
(72) Inventor: Okazaki, Kiyoshi, Shioya-gun, Tochigi-ken (JP)
(74) Representative: Blumbach, Kramer & Partner

(56) References cited:
- EP-A- 0 301 360
- EP-A- 0 316 863
- DE-A- 3 900 893
- FR-A- 2 587 493
- Lithotripsy II (Eds. M.J. Coptcoat et al.), September 1987, pages 65-88; K.M. Tomera et al.: "Sonolith 2000: Technomed International"

## Description

### BACKGROUND OF THE INVENTION

### Field of the Invention

The present invention relates to a shock wave medical treatment apparatus for treating an object to be treated inside a patient such as a cancer cell and concretion by disintegrating them by means of a focusing energy of a shock wave.

### Description of the Background Art

An example of a conventional shock wave medical treatment apparatus, an ultrasonic wave applicator disclosed in Japanese Patent Application Laid Open No. 62-049843 (1987) is shown in Fig. 1.

This ultrasonic wave applicator 1 comprises a concave oscillator 2 of 10 cm diameter and a constant curvature for generating focused ultrasonic waves which has an aperture of a prescribed shape at a middle, a backing material 3 attached to a back of this concave oscillator 2 uniformly, an imaging ultrasonic wave probe 4 for taking images of region to be treated by transmitting and receiving imaging ultrasonic waves which is fixed at the aperture of the concave oscillator 2 and which is a sector scanning type having an array 4a of minute oscillator elements at a bottom face, and an acoustic coupler 5 for propagating the focused ultrasonic waves from the concave oscillator 2 to a surface 32S of a patient which is made of a bag 6 of thin film having an acoustic impedance substantially equal to that of water and which is filled with water.

In this ultrasonic wave applicator 1, the array 4a of the oscillator elements of the imaging ultrasonic wave probe 4 is either aligned with a curved ultrasonic wave generating surface of the concave oscillator 2 or placed further away from the surface 32S of the patient than the curved ultrasonic wave generating surface of the concave oscillator 2.

In such a configuration, the imaging ultrasonic waves to be transmitted and received by the imaging ultrasonic wave probe 4 inevitably suffer from scattering and dissipation due to the intervening water inside the acoustic coupler 5 and the thin film of the bag 6. For this reason, the images to be displayed by a display unit (not shown) are influenced by noise so that locating the object to be treated becomes difficult.

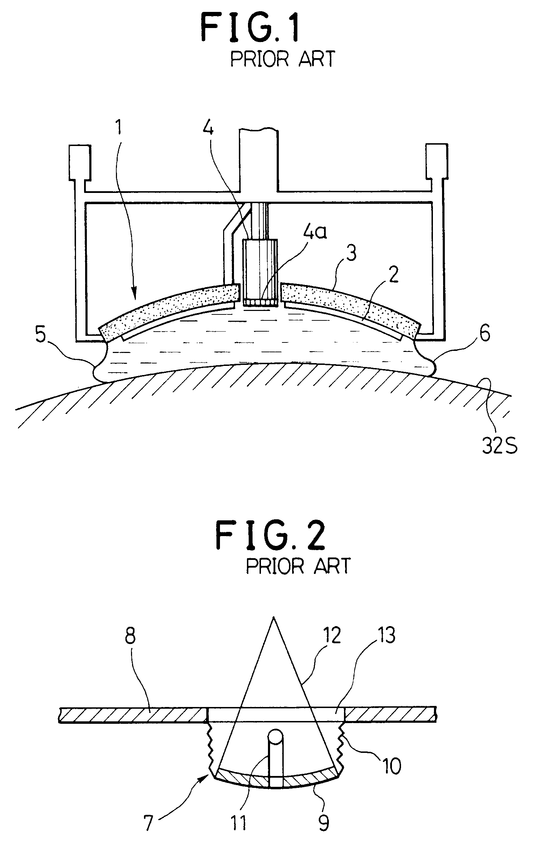

On the other hand, there is an alternative configuration for the ultrasonic wave applicator which is shown in Fig. 2.

This ultrasonic wave applicator 7 comprises a concave oscillator 9 for generating ultrasonic waves of focusing trajectory 12 which is similar to the concave oscillator 2 of the previous example described above and which is located below a bed 8, a water bag 10 filled with water for transmitting the ultrasonic waves which is provided between an ultrasonic wave generating surface of the concave oscillator 9 and an opening 13 of the bed 8 located above the concave oscillator 9, and a mechanical probe 11 placed inside the water bag 10 which has a fixed focal length.

In this alternative configuration, the problem of scattering and dissipation as described above for the previous example is also present. In addition, there are problems due to the fact that the probe 11 is a mechanical probe, such as deterioration of the image quality, enlargement of the configuration, and vulnerability against vibrations. Moreover, in a configuration of Fig. 2, operation of the ultrasonic wave applicator 7 is harder because this ultrasonic wave applicator 7 is fixed to the bed 8.

To deal with such problems of a conventional shock wave medical treatment apparatus, the present inventor has proposed a shock wave applicator comprising a shock wave transducer for generating shock waves with a focal point located inside a patient, a water bag provided at a shock wave generating surface of the shock wave transducer, and an imaging ultrasonic transducer located between the shock wave generating surface and the focal point for taking image data of the patient in a region including the focal point in a state of contacting its ultrasonic wave transmitting and receiving surface to a surface of the patient, as disclosed in Japanese Patent Application Laid Open No. 62-290158 (1987), or EP-A-0 361 863.

Now, in general, a distance from a body surface to the object to be treated varies according to an organ containing the object to be treated and depending on whether the patient is adult or child. Moreover, the dissipation of the ultrasonic wave varies depending on a physical constitution of the patient.

For these reasons, there arises a necessity to change the imaging ultrasonic wave probe to that of different characteristic in accordance with the distance of the object to be treated from the body surface and the physical constitution of the patient.

However, an exchangeability of the imaging ultrasonic wave probe has not been considered in the conventional shock wave medial treatment apparatus, so that the imaging ultrasonic wave probe could not be changed so easily.

A shock wave applicator for a shock wave medical treatment apparatus according to the preamble of the appended independent claim 1 is disclosed in EP-A-0 361 863. The housing pipe means of this known shock wave applicator is constituted by a cylindrical member, the upper end of which is bonded to the periphery of the central aperture of the shock wave transducer means and the lower end of which may be formed integrally with the bottom portion of the acoustic coupler means. The ultrasonic wave probe means is inserted into the cylindrical member constituting the housing pipe means. There is no disclosure of any possibility to change the relative position between the ultrasonic wave probe means and the shock wave transducer means. Furthermore with this known shock wave applicator any change in the relative position between the shock wave transducer means and the ultraonic wave probe means would cause a change in the distance between the ultrasonic wave probe means and the surface of the body to be treated, which might negatively influence the quality of the images generated by the ultrasonic wave probe means. A further aspect of this shock wave applicator is that the distance between the shock wave transducer means and the bottom of the acoustic coupler means, constituted by a water tank, is almost fixed by the cylindrical member, which causes a lower flexibility in adapting the apparatus to the needs of different medical treatments.

From EP-A-0 301 360 there is known a shock wave applicator with an acoustic coupler means being closed on its bottom surface. Therefore the housing pipe means, which is disposed through an aperture of the shock wave transducer means ends within the acoustic coupler means. An imaging ultrasonic wave probe means, being disposed within the housing pipe means is in direct contact with the coupling liquid within the acoustic coupler means. Due to this construction there must be provided sealing means between the housing pipe means and the shock wave transducer means and in addition sealing means between the housing pipe means and the ultrasonic wave probe means. In addition the path length of the ultrasonic waves of the wave probe means through the liquid within the acoustic coupler means varies according to the position of the ultrasonic wave probe means within the housing pipe means.

### Summary of the Invention

It is an object of the invention to provide a shock wave medical treatment apparatus in which the imaging ultrasonic wave probe can be changed among those of different imaging region depths, so that locating of the object to be treated inside the patient becomes easier, and wherein the relative position of the shock wave transducer means and the imaging ultrasonic wave probe means is adjustable.

This object is achieved by a shock wave applicator for a shock wave medical treatment apparatus with the features of claim 1 as appended.

The simple exchangeability of the ultrasonic wave probe means is due to the fact that the ultrasonic wave probe means is completely separated from the interior of the acoustic coupler means and that there is no necessity that the means for moving the ultrasonic wave probe means relative to the shock wave transducer means is engaging into any surface of the ultrasonic wave probe means.

Dependent claims 2 to 6 are directed towards advantageous embodiments of the inventive apparatus. A special advantage of the embodiments according to the claims 5 and 6 is that it is simply possible to keep the ultrasonic wave probe means to be rotatable at a fixed position with respect to the body surface while changing the relative position of the shock wave transducer means and the ultrasonic wave means.

Other features and advantages of the present invention will become apparent from the following description taken in conjunction with the accompanying drawings.

### BRIEF DESCRIPTION OF THE DRAWINGS

Fig. 1 is a schematic cross sectional view of one example of a conventional shock wave medical treatment apparatus.

Fig. 2 is a schematic cross sectional view of another example of a conventional shock wave medical treatment apparatus.

Fig. 3 is a schematic block diagram of one embodiment of a shock wave medical treatment apparatus according to the present invention.

Fig. 4 is a detailed cross sectional view of a shock wave transducer in the apparatus of Fig. 3.

Fig. 5 is an enlarged cross sectional view of a position detection unit of the shock wave transducer of Fig. 4.

Fig. 6 is an enlarged cross sectional view of a portion A of the shock wave transducer of Fig. 4.

Fig. 7 is an enlarged cross sectional view of a portion B of the shock wave transducer of Fig. 4.

Fig. 8 is an enlarged cross sectional view of a portion C of the shock wave transducer of Fig. 4.

Fig. 9 is an enlarged cross sectional view of a portion D of the shock wave transducer of Fig. 4.

Fig. 10 is an enlarged cross sectional view of an alternative configuration for the portion D shown in Fig. 9.

### DETAILED DESCRIPTION OF THE PREFERRED EMBODIMENTS

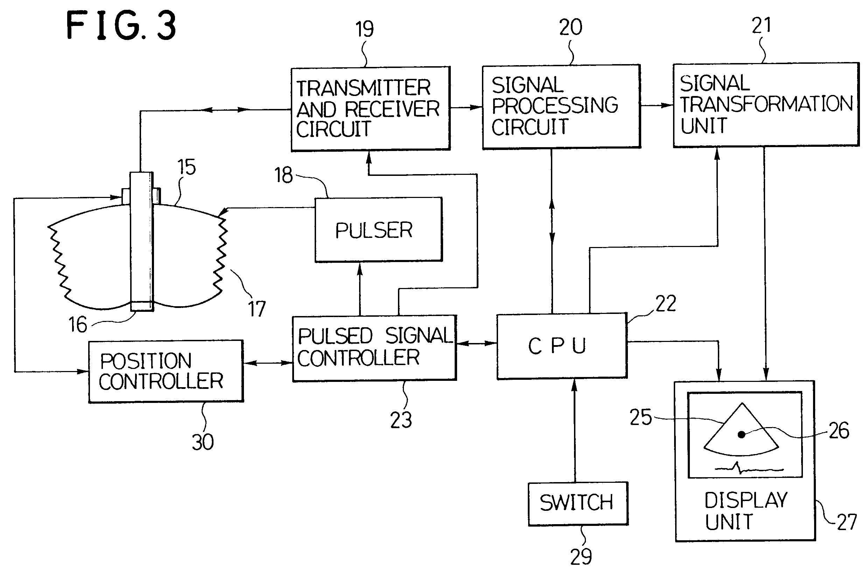

Referring now to Fig. 3, there is shown one embodiment of a shock wave medical treatment apparatus according to the present invention, in a form of an ultrasonic medical treatment apparatus.

In this embodiment, the shock wave medical treatment apparatus comprises a shock wave applicator 17 including a shock wave transducer 15 for generating ultrasonic shock waves as shock waves for treatment and an imaging ultrasonic wave probe 16 for transmitting and receiving imaging ultrasonic waves, which will be described in greater detail below, a pulser 18 for activating the shock wave transducer 15 by supplying pulsed signals, a transmitter and receiver circuit 19 for activating the imaging ultrasonic wave probe 16 into sector scanning by supplying pulsed signals and receiving echo signals obtained by the imaging ultrasonic wave probe 16 as a result of the sector scanning, a signal processing circuit 20 for converting output signals of the transmitter and receiver circuit into video signals by detecting amplitudes of the output signals, a signal transformation unit 21 such as a digital scan converter for performing signal transformation operation on output signals of the signal processing circuit 20, a CPU 22 for controlling elements of the apparatus, a pulsed signal controller 23 for controlling timings, amplitudes and frequencies of the pulsed signals at the pulser 18 and the transmitter and receiver circuit 19, a display unit 27 for displaying images such as those of a patient's body surface, an organ of interest, and concretion to be treated in the organ of interest in a fan shape imaging region 25 imaged by the imaging ultrasonic wave probe 16 along with a focal point marker 26 indicating a focal point of the ultrasonic shock waves generated by the shock wave transducer 15 and other information, a switch 29 for activating the supply of the pulsed signals by the pulser 18 to the shock wave transducer 15, and a position controller 30 for controlling a relative position of the shock wave transducer 15 with respect to the imaging ultrasonic wave probe 16.

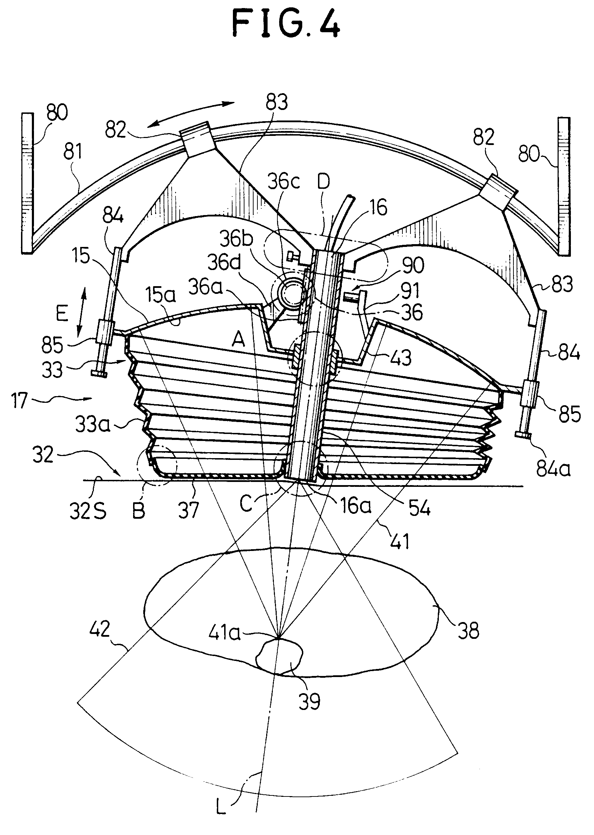

Referring now to Figs. 4 to 9, a detail configuration of the shock wave applicator 17 will be described.

As shown in Fig. 4, the shock wave applicator 17 comprises the shock wave transducer 15 for generating the ultrasonic shock wave for treating an object to be treated such as a concretion in an organ, from its constantly curved shock wave generating surface 15a equipped with concave oscillator, with the focal point 41a located inside the patient 32, which has a central aperture A, a water bag 33 as an acoustic coupler which is filled with water for propagating the ultrasonic shock waves from the shock wave transducer 15 to the patient 32 and which is attached to the shock wave transducer 15 on the shock wave generating surface 15a side, and the imaging ultrasonic wave probe 16 having an array of minute oscillator elements at a bottom face 16a for taking images in a region to be treated 42 including the focal point 41a by transmitting and receiving imaging ultrasonic waves with the bottom face 16a contacting the body surface 32S of the patient 32, which is located on a line L joining a center of the central aperture A and the focal point 41a.

In this shock wave applicator 17, the imaging ultrasonic wave probe 16 is housed inside a cylindrical housing pipe 54 made of stainless steel which has an inner diameter slightly larger than an outer diameter of the imaging ultrasonic wave probe 16, and at the central aperture A of the shock wave transducer 15 a cup shaped connecting member 43 having a slightly larger inner diameter than an outer diameter of the housing pipe 54 for supporting the shock wave transducer 15 with respect to the housing pipe 54 is provided around the housing pipe 54.

This shock wave applicator 17 is equipped with a tilting mechanism for the shock wave transducer 15 and the imaging ultrasonic wave probe 16 so that the shock wave transducer 15 and the imaging ultrasonic wave probe 16 can be tilted to a desired direction toward the patient 32. The tilting mechanism comprises a couple of symmetrically arranged connectors 83, each of which having a first guide 82 at a top which is slidably engaged to a circular guide rail 81. The circular guide rail 81 is suspended by a suspension frame 80 which can suspend the entire shock wave applicator 17, and is curved such that it has a shape of a part of circle centered around a middle of the bottom face 16a of the imaging ultrasonic wave probe 16. Each connector 83 has two bottom ends one of which is connected to a top end portion of the housing pipe 54 while another one of which is connected to a vertical guide rail 84 provided parallel to the housing pipe 54 by the shock wave transducer 15. The shock wave transducer 15 is connected with the vertical rails 84 through second guides 85 attached at an edge of the shock wave transducer 15 which are slidably engaged with the vertical guide rails 84. Each of the vertical guide rails 84 has a stopper 84a at a bottom end to prevent the second guide 85 from disengaging off the vertical guide rail 84. Thus, by sliding the first guides 82 along the circular guide rail 81 manually, the shock wave transducer 15 and the imaging ultrasonic wave probe 16 can be tilted to a desired direction toward the patient 32, without changing a relative position of the shock wave transducer 15 with respect to the imaging ultrasonic wave probe 16. Also, a vertical plane in which the tilting takes place can be rotated by rotating the suspension frame 80 around.

Furthermore, between the connecting member 43 and the housing pipe 54, there is provided a driver unit 36 for moving the shock wave transducer 15 in a direction E with respect to the housing pipe 54. This driver unit 36 comprises a rack 36a fixed on a side face of the housing pipe 54, a pinion gear 36b to be engaged with the rack 36a, a motor 36c as a source of driving power whose driving axis is connected to the pinion gear 36b, and a supporting member 36d for supporting the motor 36c with respect to the connecting member 43. The driver unit 36 moves the shock wave transducer 15 in a desired manner indicated by a control signal supplied from the position controller 30 appeared in Fig. 3.

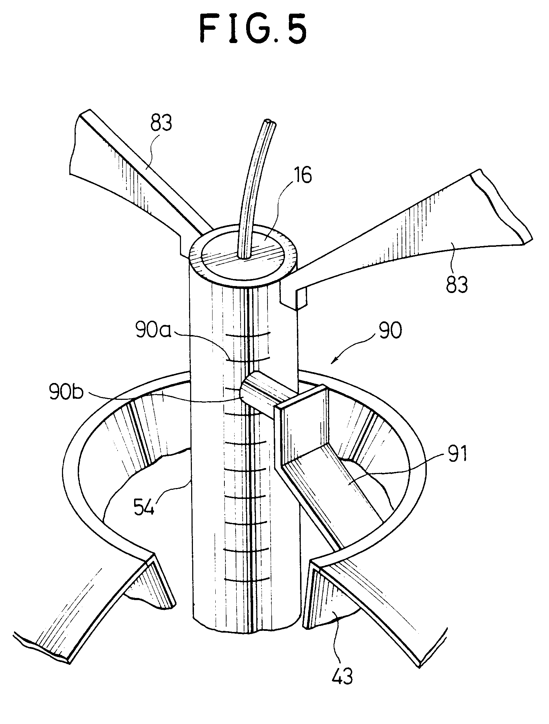

A position of the shock wave transducer 15 in the direction E with respect to the housing pipe 54 is detected by a position detection unit 90. As shown in Fig. 5, the position detection unit 90 comprises scale markings 90a provided on a side face of the housing pipe 54 for indicating prescribed intervals, a photo sensor 90b for reading the scale markings 90a, and a supporting element 91 for supporting the photo sensor 90b with respect to the connecting member 43.

The reading of the scale markings read by the photo sensor 90b is signaled to the position controller 30, which in turn controls rotation angle of the driving axis of the motor 36c such that the position of the shock wave transducer 15 with respect to the imaging ultrasonic wave probe 16 is controlled in a desired manner. When the motor 36c is not activated, the position of the shock wave transducer 15 is held fixed.

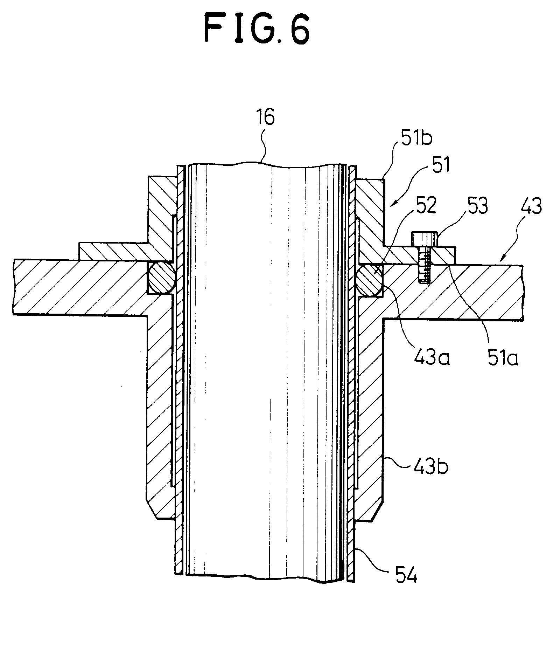

As shown in Fig. 6, in a vicinity of the central aperture A of the shock wave transducer 15, the connecting member 43 includes a lower pipe section 43b having a groove 43a for housing an O-ring 52 on an inner circumference side in contact with the housing pipe 54. The connecting member 43 is also equipped with a stopper member 51 having an upper pipe section 51b, a nut section 51a extending perpendicular to the upper pipe section 51b, and a bolt 53 to be engaged into the nut section 51a for fastening the stopper member 51 to a main part of the connecting member 43. The lower pipe section 43b and the upper pipe section 51b function to keep the housing pipe 54 from tilting with respect to the connecting member 43, while the O-ring 52 function to prevent leakage of the water contained inside the water bag 33 even when the shock wave transducer 15 is moved in the direction E.

The water bag 33 comprises a side face 33a in a form of bellows that can extend or contract in a certain range of angles around the direction E whose upper circumference is attached to the circumference of the shock wave transducer 15, and a bottom face 37 made of a thin film of rubber which has substantially equivalent acoustic impedance as the water.

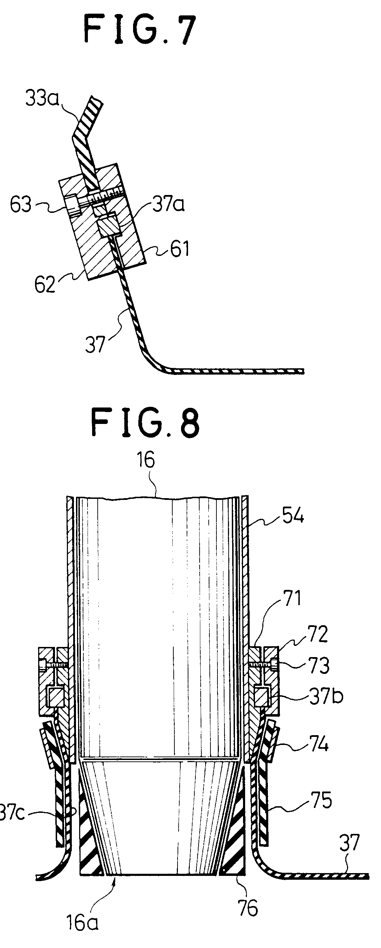

As shown in Fig 7, the bottom face 37 has an outer reinforcement ring 37a formed around its outer rim, and the side face 33a and the bottom face 37 are connected by pinching an edge of the side face 33a and the reinforcement ring 37a together in between ring shaped fittings 61 and 62 fastened by a bolt 63.

On the other hand, as shown in Fig. 8, the bottom face 37 also has an inner reinforcement ring 37b formed around its inner rim, and the bottom face 37 are fixed by pinching the reinforcement ring 37a in between ring shaped fittings 71 and 72 fastened by a bolt 73.

In addition, in order to wrap around an end portion of the imaging ultrasonic wave probe 16 with an inner side wall portion 37c of the bottom face 37 tightly, there is provided an additional reinforcement member 75 made of rubber harder than that of the bottom face 37, at the inner side wall portion 37c, which is held fixed on the inner side wall portion 37c by a band 74, and an elastic ring shaped sponge 76 attached to the end portion of the imaging ultrasonic wave probe 16.

Thus, as being made of thin film, the bottom face 37 may be ripped off, but in such a case the bottom face 37 can easily be replaced by separating the fittings 61 and 62 on the outer rim apart to remove the outer reinforcement ring 37a off, removing the water inside the water bag 33, separating the fittings 71 and 72 on the inner rim, and removing the band 74 on the inner side wall portion 37c.

Now, the imaging ultrasonic wave probe 16 has a fixed penetration depth, and this in turn determines a depth of the region to be treated. On the other hand, a distance between the body surface and the concretion differs for an adult patient and a child patient, even when the organ containing the concretion is identical. For this reason, it is preferable to use the imaging ultrasonic wave probe 16 of low frequency (3.75 MHz) for an adult patient in order to take image at deeper position, and to use the imaging ultrasonic wave probe 16 of high frequency (5 MHz) for a child patient in order to take image at shallower position. The high frequency imaging ultrasonic wave probe 16 also has an advantage of being capable to obtain high quality images. Similarly, it is preferable to select an appropriate one from more than one imaging ultrasonic wave probe 16 of different frequencies depending on depths of the concretion to be disintegrated. Also, it is preferable to select an appropriate one from more than one imaging ultrasonic wave probes 16 of different frequencies depending on physical constitution of the patient, such as the imaging ultrasonic wave probe 16 of low frequency for a muscular patient, and the imaging ultrasonic wave probe 16 of high frequency for a less muscular patient.

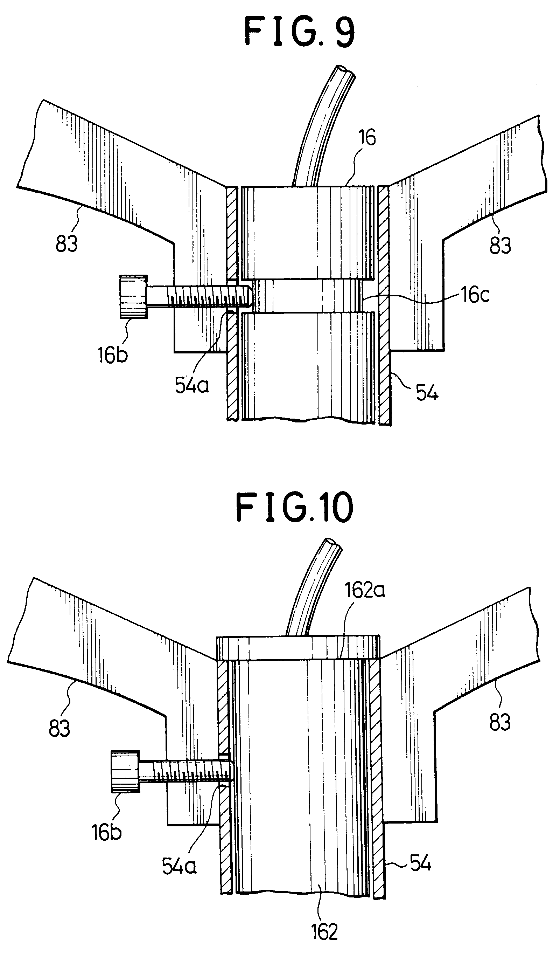

For this reason, in this embodiment, the imaging ultrasonic wave probe 16 is housed inside the housing pipe 54 so as to be easily changed by another. Moreover, in this embodiment, the imaging ultrasonic wave probe 16 is rotatable around its central axis. More specifically, as shown in Fig. 9, a clearance of about 0.5 to 1 mm is provided between the inner diameter of the housing pipe 54 and the outer diameter of the imaging ultrasonic wave probe 16 so that the imaging ultrasonic wave probe 16 can easily be inserted into the housing pipe 54. In addition, the imaging ultrasonic wave probe 16 has a groove 16c in a vicinity of its top end portion such that a bolt 16b can be fastened through a hole 54a provided on the housing pipe 54 into the groove 16c. When the bolt 16b is fully tightened the imaging ultrasonic wave probe 16 can be fixed at a desired position, whereas when the bolt 16 is only loosely tightened the imaging ultrasonic wave probe 16 can be rotated around its central axis.

Now, the operation of this embodiment of a shock wave medical treatment apparatus will be described for an exemplary case of treating a concretion 39 inside an organ 38 shown in Fig. 4.

The imaging ultrasonic wave probe 16 having an appropriate penetration depth for this concretion 39 to be treated is selected in advance, installed into the the housing pipe 54, and fixed by means of the bolt 16b.

Then, the water bag 33 of the shock wave applicator 17 is placed over the body surface 32S of the patient 32 above the organ 38, with the imaging ultrasonic wave probe 16 contacting the body surface 32S.

Next, the imaging ultrasonic wave probe 16 is activated along with the transmitter and receiver circuit 19, the signal processing circuit 20, and the signal transformation unit 21, such that a tomographic image of the patient 32 is displayed on the display unit 27. Here, because the imaging ultrasonic wave probe 16 is contacting the body surface 32S, a very clear image without influences of the water or the bottom face 37 of the water bag 33 is obtainable, and this helps a quick and easy apprehension of the concretion 39.

Next, the imaging ultrasonic wave probe 16 is operated to show the concretion 39 on the tomographic image displayed on the display unit 27. This operation may involve such maneuvering for obtaining a clearer image as tilting the imaging ultrasonic wave probe 16 by sliding the first guides 82 along the circular guiding rail 81, and rotating the imaging ultrasonic wave probe 16 around the line L by slightly loosening the bolt 16b.

On the fan shape imaging region 25 of the display unit 27, a relative position of the focal point 41a of the shock wave transducer 15 with respect to the imaging ultrasonic wave probe 16 is indicated in real time in a form of the marker 26 according to the signals from the position detection unit 90 transmitted through the CPU 22 and the signal transformation unit 21. When the concretion 39 is displayed on the tomographic image, the position controller 30 is operated to move the shock wave transducer 15 in the direction E until the marker 26 and the concretion 39 coincide on the display unit 27. Here, because the bottom face 16a of the imaging ultrasonic wave probe 16 is located on the line L joining a center of the central aperture A and the focal point 41a, the concretion 39 can be displayed at a center of the displayed image, and this also helps a quick and easy apprehension of the concretion 39. Also, because the relative position of the shock wave transducer 15 with respect to the imaging ultrasonic wave probe 16 is adjustable, the treatment operation become easier.

Next, the switch 29 is operated to send the control signal to the pulser 18 through the CPU 22 and the pulsed signal controller 23, such that the pulsed signals are transmitted from the pulser 18 to the shock wave transducer 15 and the powerful ultrasonic shock waves are generated from the shock wave transducer 15 toward the concretion 39 located at the position indicated by the marker 26 on the display unit 27 to disintegrate the concretion 39. Such a shock wave application is repeated as many times as necessary to completely disintegrate the concretion 39.

As described, in this embodiment of a shock wave medical treatment apparatus, a quick and easy apprehension of the concretion 39 is possible because a very clear image without influences of the water or the bottom face 37 of the water bag 33 is obtainable as a result of the fact that the imaging ultrasonic wave probe 16 is contacting the body surface 32S.

A clear image is obtainable also because the imaging ultrasonic wave probe 16 is rotatable around its central axis,
Moreover, the imaging ultrasonic wave probe 16 is housed inside the housing pipe 54 so that one having an appropriate penetration depth can quickly and easily be installed.

In addition, since the imaging ultrasonic wave probe 16 is completely separated from the water in the water bag 33, the damaging of the imaging elements due to wetting of the imaging elements through the lenses covering the imaging elements, which has been accompanying the change of the probe in a conventional shock wave medical treatment apparatus, can be prevented.

Also, because of the water bag 33 which functions as an acoustic coupler, the shock wave can be transmitted efficiently.

Also, because the side face of the water bag 33 are bellows, the water bag 33 can easily be stretched or contracted in a vertical direction, and the adjustment of the relative position of the shock wave transducer 15 with respect to the imaging ultrasonic wave probe 16 becomes easier. Here, the leakage of the water from the water bag 33 in moving the shock wave transducer 15 is prevented by the O-ring 52 provided between the connecting member 43 and the housing pipe 54.

Also, because the focal point 41a of the ultrasonic shock wave from the shock wave transducer 15 is indicated on the display unit 27 by the marker 26, an accurate treatment can be performed easily.

Now, there are several variations of the embodiment described above possible.

First of all, a configuration of a portion D in Fig. 4, which is shown in detail in Fig. 9, may be altered to that shown in Fig. 10. In this alternative configuration, the imaging ultrasonic wave probe 162 is equipped with a stopper 162a at its top end, which replaces the groove 16c of the above embodiment. Thus, when the imaging ultrasonic wave probe 162 is inserted into the housing pipe 54, the position of the imaging ultrasonic wave probe 162 is determined as the stopper 162a abuts the top edge of the housing pipe 54. The imaging ultrasonic wave probe 162 can be fixed, or made free to rotate by tightening or loosening the bolt 16b as in the above embodiment.

Also, the shock wave transducer 15 may be of an electromagnetic induction type shock wave generator instead of a generator using oscillator elements as in the above embodiment.

Also, the position detection unit 90 of the above embodiment may be replaced by a potentiometer of variable resistor type provided in conjunction with the pinion gear 36b.

Also, instead of moving the shock wave transducer 15, the imaging ultrasonic wave probe 16 may be made to be movable with respect to the shock wave transducer 15.

Besides these, many modifications and variations of the above embodiments may be made without departing from the novel and advantageous features of the present invention. Accordingly, all such modifications and variations are intended to be included within the scope of the appended claims.

## Claims

1. A shock wave applicator for a shock wave medical treatment apparatus, comprising:
shock wave transducer means (15) for generating shock waves with their focal point (41a) inside a body (32) to be treated, the shock wave transducer means having an aperture at its center;
imaging ultrasonic wave probe means (16) for taking images of the body (32) to be treated containing the focal point (41a) of the shock waves;
acoustic coupler means (33), arranged to be in contact with the shock wave transducer means (15), for propagating the shock waves between the shock wave transducer means (15) and the body (32) to be treated, the acoustic coupler means (33) having a central hole on a bottom surface; and
housing pipe means (54) for holding the imaging ultrasonic wave probe means (16) therein
which housing pipe means (54) is disposed through the aperture of the shock wave transducer means (15) and the central hole of the acoustic coupler means (33), placed on a line joining the focal point (41a) of the shock waves and the center of the aperture of the shock wave transducer means (15), whereby the acoustic coupler means (33) is completely separated from the imaging ultrasonic wave probe means (16) held inside the housing pipe means (54) by the housing pipe means (54),
characterized in that
means (36a, 36b, 36c, 36d) are provided for sliding the shock wave transducer means (15) along the housing pipe means (54), and that the imaging ultrasonic wave probe means (16) is exchangeably held inside the housing pipe means (54).

2. The apparatus of claim 1, wherein the means (36a, 36b, 36c, 36d) for sliding changes the relative position of the shock wave transducer means (15) with respect to the imaging ultrasonic wave probe means (16) and the body (32) to be treated.

3. The apparatus of claim 1, wherein the acoustic coupler means (33) comprises a water bag filled with water capable of stretching and contracting at least in a direction along the line joining the focal point (41a) of the shock waves and the center of the aperture of the shock wave transducer means (15).

4. The apparatus of claim 1, wherein a bottom face (16a) of the imaging ultrasonic wave probe means (16) and the bottom face (37) of the acoustic coupler means (33) form an externally exposed surface which is adapted to directly contact a surface (32S) of the body (32) to be treated.

5. The apparatus of claim 1, wherein the housing pipe means (54) holds the imaging ultrasonic wave probe means (16) to be rotatable around a central axis of the imaging ultrasonic wave probe means (16).

6. The apparatus of claim 5, wherein the housing pipe means (54) includes means (16b) for preventing the imaging ultrasonic wave probe means (16) from rotating.

## Patentansprüche

1. Stoßwellenapplikator für eine Einrichtung zur medizinischen Stoßwellenbehandlung, enthaltend:
eine Stoßwellen-Wandlervorrichtung (15) zum Erzeugen von Stoßwellen, deren Brennpunkt (41a) innerhalb eines zu behandelnden Körpers (32) liegt, welche Stoßwellen-Wandlervorrichtung in ihrem Zentrum eine Öffnung aufweist;
eine bilderzeugende Ultraschallsondenvorrichtung (16) zum Aufnehmen von Bildern des zu behandelnden Körpers (32), die den Brennpunkt (41a) der Stoßwellen enthalten;
eine Akustikkopplervorrichtung (33), die derart angeordnet ist, daß sie in Berührung mit der Stoßwellen-Wandlervorrichtung (15) ist, zum Übertragen der Stoßwellen zwischen der Stoßwellen-Wandlervorrichtung (16) und dem zu behandelnden Körper (32), welche Akustikkopplervorrichtung (33) an einer Bodenfläche ein zentrales Loch aufweist; und
eine Aufnahmerohrvorrichtung (54) zum Halten der bilderzeugenden Ultraschallsondenvorrichtung (16) in sich, welche Aufnahmerohrvorrichtung (54) durch die Öffnung der Stoßwellen-Wandlervorrichtung (15) und das zentrale Loch der Akustikkopplervorrichtung (33) hindurchgehend angeordnet ist, das sich auf einer Linie befindet, die den Brennpunkt (41a) der Stoßwellen und das Zentrum der Öffnung der Stoßwellen-Wandlervorrichtung (15) verbindet, wodurch die Akustikkopplervorrichtung (33) mittels der Aufnahmerohrvorrichtung (54) vollständig von der bilderzeugenden Ultraschallsondenvorrichtung (16) getrennt ist, die innerhalb der Aufnahmerohrvorrichtung (54) gehalten ist,
dadurch **gekennzeichnet**, daß
eine Vorrichtung (36a, 36b, 36c, 36d) zum Verschieben der Stoßwellen-Wandlervorrichtung (15) längs der Aufnahmerohrvorrichtung (54) vorgesehen ist, und daß die bilderzeugende Ultraschallsondenvorrichtung (16) auswechselbar innerhalb der Aufnahmerohrvorrichtung (54) gehalten ist.

2. Einrichtung nach Anspruch 1, wobei die Vorrichtung (36a, 36b, 36c, 36d) zum Verschieben die relative Lage der Stoßwellen-Wandlervorrichtung (15) bezüglich der bilderzeugenden Ultraschallsondenvorrichtung (16) und dem zu behandelnden Körper (32) verändert.

3. Einrichtung nach Anspruch 1, wobei die Akustikkopplervorrichtung (33) einen mit Wasser gefüllten Wasserbehälter enthält, der zumindest in einer Richtung längs der Linie ausdehnbar und zusammenziehbar ist, die den Brennpunkt (41a) der Stoßwellen mit dem Zentrum der Öffnung der Stoßwellen-Wandlervorrichtung (15) verbindet.

4. Einrichtung nach Anspruch 1, wobei eine Bodenfläche (16a) der bilderzeugenden Ultraschallsondenvorrichtung (16) und die Bodenfläche (37) der Akustikkopplervorrichtung (33) eine extern freiliegende Fläche bilden, die in direkte Berührung mit einer Oberfläche (32S) des zu behandelnden Körpers (32) bringbar ist.

5. Einrichtung nach Anspruch 1, wobei die Aufnahmerohrvorrichtung (54) die bilderzeugende Ultraschallsondenvorrichtung (16) um eine zentrale Achse der bilderzeugenden Ultraschallsondenvorrichtung (16) drehbar hält.

6. Einrichtung nach Anspruch 5, wobei die Aufnahmerohrvorrichtung (54) eine Vorrichtung (16b) zum Hindern der Drehung der bilderzeugenden Ultraschallsondenvorrichtung (16) enthält.

## Revendications

1. Applicateur d'ondes de choc pour un appareil de traitement médical à ondes de choc, comprenant :
- des moyens formant émetteur d'ondes de choc (15) pour produire des ondes de choc avec un foyer (41a) à l'intérieur d'un corps (32) à traiter, les moyens formant émetteur d'ondes de choc ayant une ouverture à leur centre ;
- des moyens formant capteur d'imagerie à ondes ultrasonores (16) pour prendre des images du corps (32) à traiter qui contient le foyer (41a) des ondes de choc ;
- des moyens formant coupleur acoustique (33), agencés de manière à être en contact avec les moyens formant émetteur d'ondes de choc (15), pour propager les ondes de choc entre les moyens formant émetteur d'ondes de choc (15) et le corps (32) à traiter, le coupleur acoustique (33) ayant un trou central sur une surface inférieure ; et
- des moyens formant tube de boîtier (54) pour y maintenir les moyens formant capteur d'imagerie à ondes ultrasonores (16), lesdits moyens formant tube de boîtier (54) étant disposés à travers l'ouverture des moyens formant émetteur d'ondes de choc (15) et le perçage central des moyens formant coupleur acoustique (33), placé sur une ligne qui rejoint le foyer (41 a) des ondes de choc et le centre de l'ouverture des moyens formant émetteur d'ondes de choc (15), grâce à quoi les moyens formant coupleur acoustique (33) sont entièrement séparés, par les moyens formant tube de boîtier (54), des moyens formant capteur d'imagerie à ondes ultrasonores (16) maintenus à l'intérieur des moyens formant tube de boîtier (54)
caractérisé en ce que
- des moyens (36a, 36b, 36c, 36d) sont prévus pour faire coulisser les moyens formant émetteur d'ondes de choc (15) le long des moyens formant tube de boîtier (54), et
- en ce que les moyens formant capteur d'imagerie à ondes ultrasonores (16) sont maintenus de manière interchangeable à l'intérieur des moyens formant tube de boîtier (54).

2. Appareil selon la revendication 1 dans lequel les moyens (36a, 36b, 36c, 36d) destinés au coulissement modifient la position relative des moyens formant émetteur d'ondes de choc (15) par rapport aux moyens formant capteur d'imagerie à ondes ultrasonores (16) et au corps (32) à traiter.

3. Appareil selon la revendication 1, dans laquelle les moyens formant coupleur acoustique (33) comprennent un sac à eau rempli d'eau, capable de s'allonger et de se contracter au moins dans une direction le long de la ligne qui joint le foyer (41a) des ondes de choc et le centre de l'ouverture des moyens formant émetteur d'ondes de choc (15).

4. Appareil selon la revendication 1, dans lequel une face inférieure (16a) des moyens formant capteur d'imagerie à ondes ultrasonores (16) et la face inférieure (37) des moyens formant coupleur acoustique (33) forment une surface exposée vers l'extérieur, qui est adaptée à venir directement en contact avec une surface (32S) du corps (32) à traiter.

5. Appareil selon la revendication 1, dans lequel les moyens formant tube de boîtier (54) maintiennent les moyens formant capteur d'imagerie à ondes ultrasonores (16) de manière à pouvoir tourner autour d'un axe central des moyens formant capteur d'imagerie à ondes ultrasonores (16).

6. Appareil selon la revendication 5, dans laquelle les moyens formant tube de boîtier (54) comprennent des moyens (16b) pour empêcher la rotation des moyens formant capteur d'imagerie à ondes ultrasonores (16).
